# EUROPEAN PATENT APPLICATION

(11) **EP 4 470 600 A1**
(43) Date of publication of application: **04.12.2024**
(21) Application number: 22923343.2
(22) Date of filing: 20.10.2022
(51) Int. Cl.: A61N 1/05, A61N 1/36

(54) **SLICED STIMULATION ELECTRODE AND STIMULATION SYSTEM**

(30) Priority: 25.01.2022 CN 202220209369 U
(71) Applicant: SceneRay Co., Ltd., Jiangsu 215123 (CN)
(72) Inventor: JIANG, Chuanjiang, Suzhou, Jiangsu 215123 (CN)
(74) Representative: Cabinet Beaumont
(86) International application number: PCT/CN2022/126473
(87) International publication number: WO 2023/142544

(57) **Abstract**

Provided are a sliced stimulation electrode and a stimulation system. The sliced stimulation electrode includes a stimulating end and a connecting end which are opposite to each other, where multiple electrode sheets for applying electrical stimulation are disposed on a periphery surface of the stimulating end of the sliced stimulation electrode, multiple electrode sheets passing through the same radial plane of the sliced stimulation electrode form a group of electrode sheets, and in at least one group of electrode sheets, at least one electrode sheet has a different shape from the other electrode sheets so that multiple electrode sheets in the at least one group of electrode sheets can be distinguished from each other. Multiple electrode sheets in a group of electrode sheets are distinguished from each other, so it is convenient for medical personnel to quickly mark and recognize the electrode orientation through the imaging technique in use, and the electrode sheets of the stimulation electrode can be accurately aimed at the to-be-stimulated part without the need of adding electrode markers.

## Description

This application claims priority to Chinese patent application No. 202220209369.3 filed Jan. 25, 2022, the disclosure of which is incorporated herein by reference in its entirety.

### TECHNICAL FIELD

The present application relates to the technical field of stimulation electrodes, in particular, to a sliced stimulation electrode and a stimulation system.

### BACKGROUND

Deep brain stimulation (DBS) involves delivering electrical stimulation to the nervous structure of specific regions in the brain to stimulate or inhibit activities of cells. DBS can effectively treat chronic pain, motor disorders such as Parkinson's disease and essential tremor, epilepsy, as well as mental illnesses such as depression and obsessive-compulsive disorder. Specifically, a stimulation electrode used for applying electrical stimulation acts on the head of the patient and stimulates a specific part of the brain, providing therapeutic effects on the brain damage of the patient. Moreover, the other end of the stimulation electrode is connected to a nerve stimulator through an electrode lead. At present, to accurately implant the electrode in the desired part of the brain and avoid side effects on other parts of the brain, the electrode lead is usually implanted relatively accurately in the desired part of the brain using various imaging techniques, such as magnetic resonance imaging (MRI), computed tomography (CT), X-ray imaging, fluorescence imaging and stereo imaging.

In most applications, it is desired to precisely place and orient the stimulation electrode in the body of the patient (such as the brain of the patient) to deliver electrical stimulation to the desired part and avoid side effects. In some applications, it is desired to locate the stimulation electrode to a place so that stimulation is delivered to a very small target point position without stimulating adjacent brain tissues. If the stimulation is not accurately delivered to the desired target point position, the efficacy may be reduced, and adjacent regions may receive unnecessary excessive stimulation. Therefore, the capability of accurately placing and locating the stimulation electrode is desired to be improved clinically.

Chinese patent CN104703653B discloses the implantation of a directional lead guided by microelectrode recording. The patent provides a method for implanting a lead into the brain tissues of the patient, where the lead includes a radial segmented electrode group at the distal end of the lead. In this method, multiple microelectrode recordings are performed on multiple recording channels respectively in the brain tissues; three-dimensional mapping of the brain structure is generated based on the microelectrode recordings; a diagram of radial segmented electrodes is located on the mapping of the brain structure so that a graphical description of the desired depth and the desired radial orientation of a lead in the brain tissues is generated; and the lead is implanted into the brain tissues according to the desired depth and the desired radial orientation. A device used for determining the radial orientation of the lead includes a radial directional scale and an indicator indicating that which electrode forms contact with the scale. To locate the electrode, the patent still uses an additional radial directional scale in conjunction with the electrode to calculate the position of the electrode, thus having certain limitations.

Chinese patent CN112292176A discloses an implantable medical lead indicator. In the patent, an electrode is disposed at the distal part of the lead and is configured to monitor a target site or provide therapy to the target site. The lead may include a visible indicator that is visible to the naked eye of the clinician at the middle part of the lead, and the visible indicator is configured to indicate that when the electrode of the lead is properly aimed at the target site longitudinally and radially for monitoring or treating the target site. The clinician can insert the lead into the body of the patient through the steps described below. An introducer sheath inserted into the body of the patient at a predetermined depth is used, and then the distal part of the lead is aligned by orienting the indicator at an entrance of the introducer sheath. The patent still adds an indicator on the lead for helping in determining the orientation of the electrode during use, thus having certain limitations.

In the existing art, a marker is generally added to the stimulation electrode for recognizing the orientation of the electrode, and the position and the orientation of the electrode is determined through corresponding relationships between predefined marker directions and the electrode stimulator. This manner requires the doctor to have a strong logical judgment ability and does not allow errors during the process, which requires too much experience from the doctor, thus having significant limitations.

Therefore, the existing stimulation electrode still needs improvement.

### SUMMARY

The present application provides a sliced stimulation electrode and a stimulation system for solving the preceding problems.

The object of the present application is implemented through the technical solutions below.

A sliced stimulation electrode is provided. The sliced stimulation electrode includes a stimulating end and a connecting end which are opposite to each other, where multiple electrode sheets for applying electrical stimulation are disposed on a periphery surface of the stimulating end of the sliced stimulation electrode, multiple electrode sheets passing through the same radial plane of the sliced stimulation electrode form a group of electrode sheets, and in at least one group of electrode sheets, at least one electrode sheet has a different shape from the other electrode sheets so that multiple electrode sheets in the at least one group of electrode sheets can be distinguished from each other

Electrode sheets having different shapes may generate different physiological efficacy. When a significant difference exists in shapes or areas of electrode sheets, different electrode sheet combinations may be designed according to the case where specific targets need special stimulation. In this manner, not only the purpose of position recognition of electrode sheets can be achieved, but also specific stimulation can be applied to specific to-be-stimulated points, so the treatment of specific diseases is achieved.

In some possible manners, the shape of each electrode sheet of the multiple electrode sheets is different, or the shape of each electrode sheet in the group of electrode sheets is different. Different sliced stimulation electrodes may be distinguished from each other according to the different shape of each electrode sheet or according to the shape of each electrode sheet of a group of electrode sheets. The two manners for marking the sliced stimulation electrode are convenient for medical personnel to choose during actual use.

In some possible manners, in a flattened state of the stimulating end of the sliced stimulation electrode, the multiple electrode sheets are arranged in an array and at intervals, and a guide shape is formed on at least one electrode sheet in the group of electrode sheets, where the guide shape of the at least one electrode sheet is used for distinguishing between multiple electrode sheets in the group of electrode sheets. The guide shape may be an arrow, a protrusion or an arc-shaped edge provided on a side of the electrode sheet. When the guide shape is actually formed on the electrode sheet, at least two electrode sheets can be quickly marked. Then, under the imaging technique, the operator can quickly and directly determine the positions of the other electrode sheets through the positions of the two marked electrode sheets, which is convenient for the operator to quickly and accurately insert the stimulation electrode into a to-be-stimulated point.

In some possible manners, the guide shape of the at least one electrode sheet is located on a side edge of the at least one electrode sheet or two opposite side edges of the at least one electrode sheet. For example, the left edge or the right edge of the electrode sheet has a guide shape, or one of the left position of the upper edge, the right position of the upper edge, the left position of the lower edge and the right position of the lower edge of the electrode sheet has a guide shape. Under the imaging technique, the positions of at least two electrode sheets can be directly marked through the guide shape, and thus the positions of the other electrode sheets are determined through comparison.

In some possible manners, the guide shape of the at least one electrode sheet is formed by a wave, a sine curve, a semicircle, a rounded rectangle or a rounded triangle, the guide shape of the at least one electrode sheet is smoothly connected to the at least one electrode sheet, and the shape of a peripheral edge of each electrode sheet is a closed curve. The guide shape may also be an arc-shaped curve edge through which at least two electrode sheets can be quickly marked, such as a wavy arc-shaped curve edge or a regular sine or cosine curve. The positions of at least two electrode sheets can be visually distinguished through a wavy edge. When electrode sheets are processed, the electrode sheets are mostly formed using the integrated molding technology. When a guide shape is provided on an electrode sheet, the steps for processing the electrode sheet can be appropriately reduced when the integrated-molded electrode sheet is processed, and thus the efficiency of processing the electrode sheet can be improved.

In some possible manners, the shape of the peripheral edge of each electrode sheet is a smooth contour curve. When the peripheral edge of the electrode sheet is a smooth curve, the smooth curve can ensure that when the stimulation electrode is inserted into the brain, no concentration or spike of stimulation signals will appear when the electrode sheet applies stimulation, and thus damage to the tissues during the stimulation can be reduced.

In some possible manners, in the flattened state of the stimulating end of the sliced stimulation electrode, the multiple electrode sheets are arranged in an array and at intervals, and a row of electrode sheets in the length direction of the sliced stimulation electrode have the same shape. When electrode sheets in the vertical direction of the sliced stimulation electrode have the same shape, medical personnel can also visually distinguish the position of the sliced electrode in the vertical direction under the imaging technique and finally determine the position of each electrode sheet in combination with the position of the sliced electrode in the radial direction of the stimulation electrode, which is convenient for medical personnel to accurately fix electrode sheets to to-be-stimulated points of the brain.

In some possible manners, the group of electrode sheets includes two to ten electrode sheets. A group of electrode sheets are a circle of electrode sheets on the same stimulation electrode. The circle of electrode sheets may include tow to ten electrode sheets. The number of electrode sheets between two to ten can help in effectively maintaining the good stimulation effect of the stimulation electrode and is convenient for the operator to observe and distinguish under the imaging technique, so most stimulation needs for neural electrical stimulation can be met.

In some possible manners, in the group of electrode sheets, the local thickness of at least one electrode sheet is different from the thickness of the other part of the at least one electrode sheet. On the same electrode sheet, if the local thickness is less than the thickness of the other part, under the imaging technique, the color of the thicker position is darker. When multiple electrode sheets work, the thickness of the electrode sheets may be used for distinguishing. During imaging of an X-ray machine, the electrode sheets can present different shades of color depending on different local thickness. Distinguishing between different electrode sheets through the shades of color is combined with distinguishing between different electrode sheets through shapes, so multiple electrode sheets can be distinguished from each other more quickly, which is convenient for medical personnel to adjust the orientation of the stimulation electrode to effectively stimulate the to-be-tested part and thus prevent the negative effect of the stimulation electrode on the brain.

A stimulation system is provided. The stimulation system includes a stimulator, a wire and a stimulation electrode, where the stimulator is connected to the stimulation electrode through the wire, and the stimulation electrode is the preceding sliced stimulation electrode.

Compared with the existing art, the present application has the beneficial effects described below.

Multiple electrode sheets in a group of electrode sheets of the sliced stimulation electrode are distinguished from each other, and different stimulation electrodes are distinguished from each other through the recognition of different shapes of electrode sheets under the imaging technique, which is convenient for the operator to quickly mark and recognize the electrode orientation and accurately apply the stimulation point of the stimulation electrode to the specified position. In this manner, the side effects caused by the inaccurate stimulation position of the stimulation electrode in the brain can be effectively reduced, and the requirement for the capability of the operator of recognizing the stimulation electrode is lowered. Moreover, it is not necessary to design additional markers for recognizing the orientation of the stimulation electrode.

### BRIEF DESCRIPTION OF DRAWINGS

FIG. 1 is a diagram of a stimulating end of a sliced stimulation electrode in a flattened state according to an embodiment of the present application;
FIG. 2 is another diagram of a stimulating end of a sliced stimulation electrode in a flattened state according to an embodiment of the present application;
FIG. 3 is another diagram of a stimulating end of a sliced stimulation electrode in a flattened state according to an embodiment of the present application; and
FIG. 4 is another diagram of a stimulating end of a sliced stimulation electrode in a flattened state according to an embodiment of the present application.

### Reference list

- 1: electrode lead
- 2: electrode sheet
- 3: sliced stimulation electrode

### DETAILED DESCRIPTION

The present application will be further described in detail in conjunction with drawings and specific embodiments. It is to be noted that the embodiments or technical features described below may be freely combined to form a new embodiment on the premise of not conflicting each other.

In the present application, "at least one" refers to one or more and "multiple" refers to two or more. "And/or" is used for describing an association between associated objects and indicates three relations, for example, "A and/or B" may indicate the presence of A alone, the presence of B alone and the presence of both A and B, where A and B may be in the singular or plural. The character "/" generally indicates an "or" relation between associated objects. "At least one of the following" or a similar expression thereof refers to any combination of items, including any combination of singular items or plural items. For example, at least one of a, b or c may indicate "a", "b", "c", "a and b", "a and c", "b and c" or "a and b and c", where a, b and c may be in the singular or plural. It is to be noted that "at least one" may also be interpreted as "one or more".

In the present application, words such as "exemplarily" or "for example" are used for expressing examples, example illustrations or explanations. In the present application, any embodiment or design scheme described as "exemplarily" or "for example" should not be construed as being more preferred or having more advantages than other embodiments or design schemes. To be exact, the use of words such as "exemplarily" or "for example" aims to present relevant concepts in a concrete manner.

Below, one field (that is, implantable medical devices) of the application fields of embodiments of the present application is briefly illustrated.

An implantable neural stimulation system (an implantable medical system) mainly includes a stimulator (that is, an implantable stimulator) implanted into the body of the patient and a program-controlled device set outside the patient. The existing neural regulation technology mainly involves that electrodes are implanted into specific structures (that is, targets) in the body through stereotactic operations, and a stimulator implanted in the body of the patient emits electrical pulses to the targets through the electrodes to regulate the electrical activities and functions of the corresponding neural structures and networks, thereby improving symptoms and alleviating pain. The stimulator may be any one of an implantable neural electrical stimulation device, a cardiac implantable electronic device (CIED, also referred to as a cardiac pacemaker), an implantable drug delivery system (IDDS) and a lead adapter device. The implantable neural electrical stimulation device is, for example, a deep brain stimulation (DBS) system, an implantable cortical nerve stimulation (CNS) system, an implantable spinal cord stimulation (SCS) system, an implantable sacral nerve stimulation (SNS) system and an implantable vagus nerve stimulation (VNS) system.

The stimulator may include an implantable pulse generator (IPG), an extension wire and an electrode lead 1. IPG is located in the body of the patient and is configured to receive program-controlled instructions from the program-controlled device. The stimulator provides controllable electrical stimulation energy to the body tissues through sealed batteries and circuits and delivers one or two routes of controllable specific electrical stimulation to specific regions of the body tissues through the implanted extension wire and the electrode lead 1. Moreover, the extension wire is used in conjunction with IPG as a transmission medium for electrical stimulation signals, transmitting the electrical stimulation signals generated by IPG to the electrode lead 1. The electrode lead 1 delivers electrical stimulation to specific regions of the body tissues through multiple electrode contacts. One or more routes of electrode leads 1 are disposed on one or two sides of the stimulator, and multiple electrode contacts are provided on the electrode lead 1. The electrode contacts may be evenly or unevenly arranged on the circumference of the electrode lead 1. In an example, the electrode contacts may be arranged in an array of four rows and three columns (a total of twelve electrode contacts) on the circumference of the electrode lead 1. The electrode contacts may include stimulation electrode contacts and/or collecting electrode contacts. The electrode contacts may be, for example, in the shape of sheets, rings and dots.

In some possible implementations, the stimulated body tissues may be the brain tissues of the patient, and the stimulated part may be a specific part of the brain tissues. When the type of the patient's disease is different, the stimulated part is generally different, and the number of stimulating contacts (single-source or multiple-source stimulating contacts) used, the number of routes of specific electrical stimulation signals (single-channel stimulation signals or multiple-channel stimulation signals) and stimulation parameter data are also different. The embodiments of the present application do not limit the applicable types of diseases, such as diseases which can be managed by using deep brain stimulation (DBS), spinal cord stimulation (SCS), pelvic stimulation, gastric stimulation, peripheral nerve stimulation and functional electrical stimulation. DBS may be used for treating or managing various types of diseases, including but not limited to, seizure disorders (such as epilepsy), pain, migraines, psychiatric disorders (such as major depressive disorder (MDD)), bipolar disorder, anxiety disorders, post-traumatic stress disorder, mild depression, obsessive-compulsive disorder (OCD), behavioral disorders, mood disorders, memory disorders, psychological disorders, movement disorders (such as essential tremors or Parkinson's disease), Huntington's disease, Alzheimer's disease, drug addiction, autism or other neurological or psychiatric disorders and impairment.

In the embodiment of the present application, when program-controlled connection between the program-controlled device and the stimulator is established, the program-controlled device may be used for adjusting stimulation parameters (different stimulation parameters correspond to different electrical stimulation signals) of the stimulator, the stimulator may be used for sensing bioelectric activities in the deep brain of the patient to collect electrophysiological signals, and the stimulation parameters of the electrical stimulation signals of the stimulator may be further adjusted through the collected electrophysiological signals.

The stimulation parameters may include at least one of: a frequency (for example, the number of electrical stimulation pulse signals per unit time of one second, in Hz), a pulse width (duration of each pulse, in µs), amplitude (generally expressed in voltage, that is, the intensity of each pulse, in V), a timing (for example, continuous or triggered timing), a stimulation mode (including one or more of a current mode, a voltage mode, a regular stimulation mode and a cyclic stimulation mode), upper and lower limits controlled by the doctor (an adjustable range by the doctor) and upper and lower limits controlled by the patient (an adjustable range by the patient).

In a specific application scenario, the stimulation parameters of the stimulator may be adjusted in the current mode or the voltage mode.

The program-controlled device may be a doctor program-controlled device (that is, a program-controlled device used by the doctor) or a patient program-controlled device (that is, a program-controlled device used by the patient). The doctor program-controlled device may be, for example, a smart end device equipped with program-controlled software such as a tablet, a laptop, a desktop or a mobile phone. The patient program-controlled device may be, for example, a smart end device equipped with program-controlled software such as a tablet, a laptop, a desktop or a mobile phone, or may be another electronic device with the program-controlled function (such as a charger with the program-controlled function or data acquisition equipment with the program-controlled function).

The embodiment of the present application does not limit the data interaction between the doctor program-controlled device and the stimulator. When the doctor performs remote program control, the doctor program-controlled device can perform data interaction with the stimulator through a server and the patient program-controlled device. When the doctor performs program control offline with the patient face to face, the doctor program-controlled device can perform data interaction with the stimulator through the patient program-controlled device, and the doctor program-controlled device can also perform direct data interaction with the stimulator.

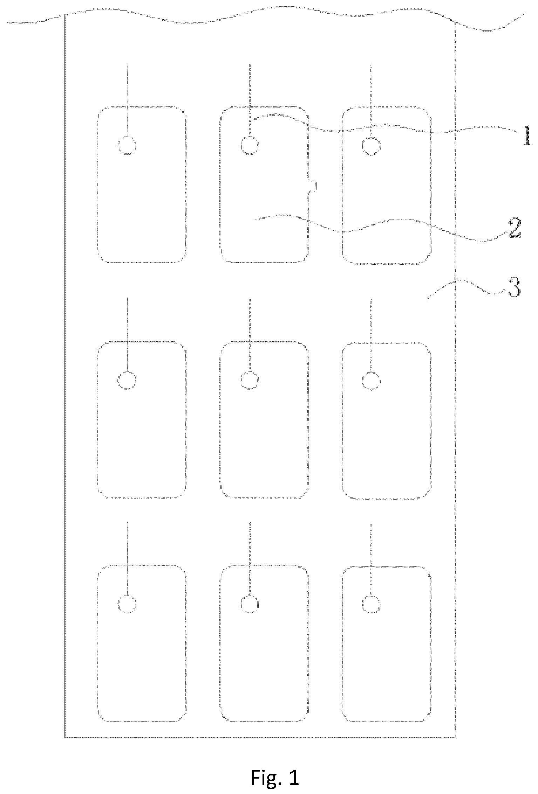

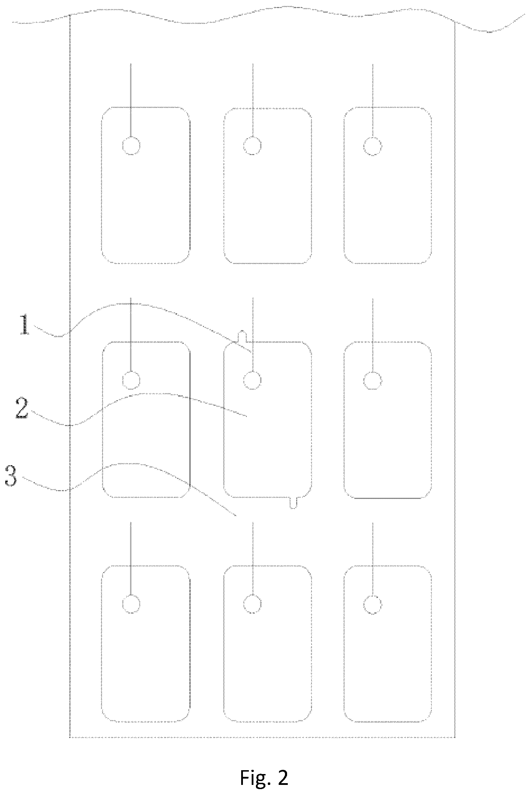

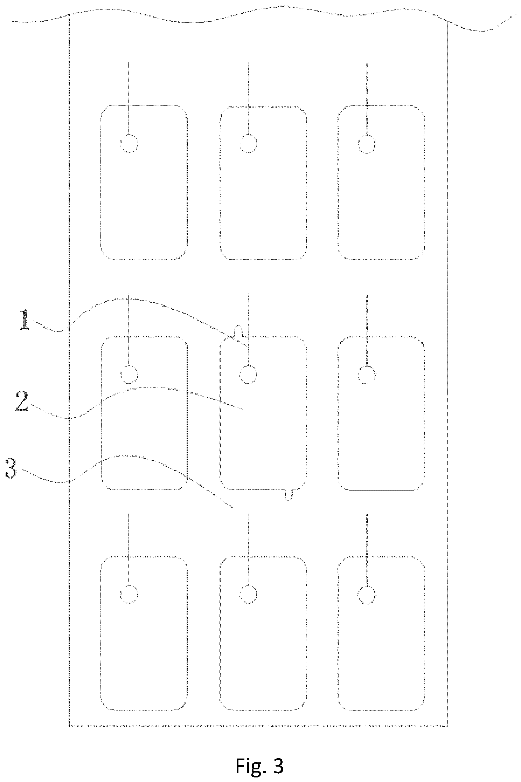

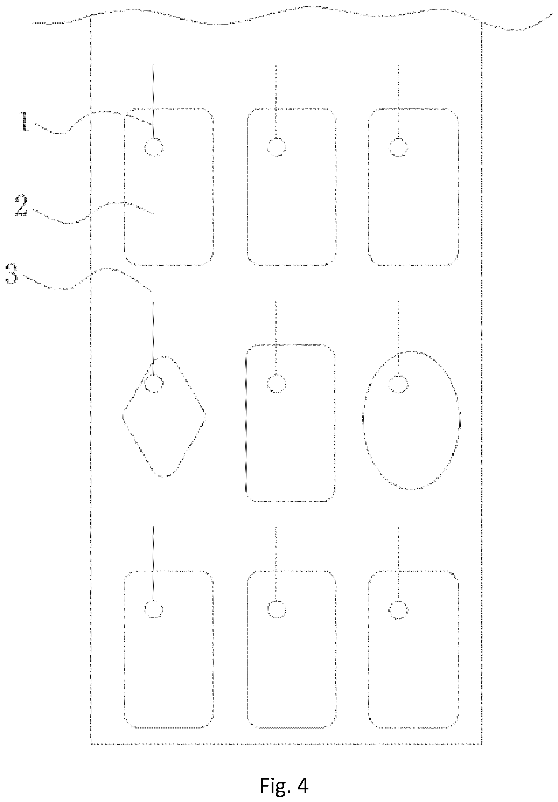

In a possible implementation, the patient program-controlled device may include a host (communicating with the server) and a slave (communicating with the stimulator), and the host is communicatively connected to the slave. The doctor program-controlled device may perform data interaction with the server through the third generation (3G)/fourth generation (4G)/fifth generation (5G) cellular network, the server may perform data interaction with the host through the 3G/4G/5G cellular network, the host may perform data interaction with the slave through the Bluetooth protocol/Wi-Fi protocol/universal serial bus (USB) protocol, the slave may perform data interaction with the stimulator through the operating frequency range of 401 MHz to 406MHz/the operating frequency range of 2.4 GHz to 2.48 GHz, and the doctor program-controlled device may perform direct data interaction with the stimulator through the operating frequency range of 401 MHz to 406MHz/the operating frequency range of 2.4 GHz to 2.48 GHz. Referring to FIG. 1 to FIG. 4, the present application provides a sliced stimulation electrode 3. The sliced stimulation electrode 3 includes a stimulating end and a connecting end which are opposite to each other. Multiple electrode sheets 2 for applying electrical stimulation are disposed on a periphery surface of the stimulating end of the sliced stimulation electrode 3. Multiple electrode sheets 2 passing through the same radial plane of the sliced stimulation electrode 3 form a group of electrode sheets 2. The group of electrode sheets 2, for example, are multiple electrode sheets 2 that surround the sliced stimulation electrode 3 in a circle in the same height direction of the sliced stimulation electrode 3. In at least one group of electrode sheets 2, at least one electrode sheet 2 has a different shape from the other electrode sheets 2 so that multiple electrode sheets 2 in the at least one group of electrode sheets 2 can be distinguished from each other. The strength, frequencies and action positions of signals output by different sliced stimulation electrodes 3 are specified, so as to avoid slight deviations generating a huge impact on the brain and other stimulated parts. When the sliced stimulation electrode 3 of the present application is used and in the process of implanting the sliced stimulation electrode 3, under the imaging technique, the different shapes of multiple electrode sheets 2 in at least one group of electrode sheets 2 are distinguished from each other, then the positions of electrode sheets 2 in other groups are distinguished, and electrode orientations are marked and recognized. The position and direction for implanting the sliced stimulation electrode 3 are determined through the electrode sheets 2 which are distinguished, which facilitates the operator to easily recognize the sliced stimulation electrode 3 and accurately fix the sliced stimulation electrode 3 to a to-be-stimulated point or a desired position of the brain without the need of adding markers on the sliced stimulation electrode 3.

Preferably, the shape of each electrode sheet 2 of the multiple electrode sheets 2 is different, or the shape of each electrode sheet 2 in the group of electrode sheet 2 is different. On the same sliced stimulation electrode 3, the shapes of all electrode sheets 2 may be completely different. In this manner, it is not necessary to distinguish positions of electrode sheets 2 in other groups according to the group of electrode sheets 2, and under the imaging technique, it is more convenient to mark and recognize the electrode orientation. Alternatively, the shape of each electrode plate 2 in the group of electrode sheets 2 may be different, so it is easy to distinguish between electrode sheets 2 in the group of electrode sheets, and then distinguish the positions of the electrode sheets 2 in other groups according to the electrode sheets 2 which are distinguished.

Referring to FIG. 1, multiple electrode sheets 2 are fixed to the sliced stimulation electrode 3, and the multiple electrode sheets 2 are connected to the connecting end of the sliced stimulation electrode 3 through electrode leads 1 and then connected to an output terminal of the external nerve stimulator through the connecting end of the sliced stimulation electrode 3.

Preferably, in a flattened state of the stimulating end of the sliced stimulation electrode 3, that is, in the state where the stimulating end of the sliced stimulation electrode 3 is unfolded and flattened, the multiple electrode plates 2 are arranged in an array and at intervals, and a guide shape is formed on at least one electrode sheet 2 in the group of electrode sheets 2, where the guide shape of the at least one electrode sheet 2 is used for distinguishing between the electrode sheets 2 in the group of electrode sheets 2. The multiple electrode sheets 2 are, preferably, evenly distributed at equal intervals on an unfolded surface of the sliced stimulation electrode 3, and the group of electrode sheets 2 become a horizontal row of electrode sheets 2 when the stimulating end of the sliced stimulation electrode 3 is in a flattened state. At least one electrode sheet 2 in the group of electrode sheets 2 has a guide shape. In simple terms, the guide shape refers to a shape with guidance. The guide shape may be an arrow, an arc-shaped protrusion or a square. The guide shape preferably has a smooth edge, and these guide shapes are, preferably, integrally molded with electrode sheets 2. The guide shape is formed on the electrode sheet 2, and when the electrode sheet 2 works, the guide shape probably does not affect the even distribution of the overall magnetic field intensity of the electrode sheet 2.

More specifically, the guide shape of the electrode sheet 2 is located on a side edge of the electrode sheet 2 or two opposite side edges of the electrode sheet 2. The guide shape of the electrode sheet 2 may be provided on any one of the upper edge, the lower edge, the left edge and the right edge of the electrode sheet 2. When the electrode sheet 2 is powered on and works, the guide shape does not affect the distribution of the magnetic field intensity of the sliced electrode. In this manner, the guide shape of the electrode sheet 2 is provided on a side edge or two opposite side edges of the electrode sheet 2; on the one hand, it is convenient for distinguishing between the positions of electrode sheets 2 in the group of electrode sheets 2 through the guide shape, and on the other hand, the impact on the distribution of the magnetic field intensity of the sliced electrode can be reduced.

Preferably, the guide shape of the electrode sheet 2 is formed by a wave, a sine curve, a semicircle, a rounded rectangle or a rounded triangle, the guide shape of the electrode sheet 2 is smoothly connected to the electrode sheet 2, and the shape of a peripheral edge of each electrode sheet 2 is a closed curve. The guide shape of the electrode sheet 2 may also be a wavy edge, a sine curve edge or a cosine curve edge which is formed on the electrode sheet 2. When a group of electrode sheets 2 include three electrode sheets 2, an edge of an electrode sheet 2 of the three electrode sheets 2 has a guide shape, so the other two electrode sheets 2 can be quickly distinguished through the position of the guide shape. In this manner, different stimulation electrodes can be simply distinguished from each other, which facilitates accurately marking and recognizing the electrode orientation and accurately implanting the sliced stimulation electrode 3 into the desired part of the brain under the imaging technique. The guide shape is formed on an arc-shaped side edge of the electrode sheet 2. When the electrode sheet 2 works, the guide shape probably does not affect the distribution of the magnetic field of the electrode sheet 2. The guide shape is designed and formed under the premise of the uniform magnetic field.

The shape of the peripheral edge of each electrode sheet 2 is a smooth contour curve. The edge of the electrode sheet 2 is a smooth curve, which ensures that when the stimulation electrode is inserted into the brain, no concentration or spike of stimulation signals will appear when the electrode sheet 2 applies stimulation, and thus damage to the tissues during the stimulation can be reduced.

In the flattened state of the stimulating end of the sliced stimulation electrode 3, the multiple electrode sheets 2 are arranged in an array and at intervals, and a row of electrode sheets 2 in the length direction of the sliced stimulation electrode 3 have the same shape. In an unfolded state, it is set that a row of electrode sheets 2 in the length direction of the multiple electrode sheets 2 have the same shape, so multiple electrode sheets 2 in each group of electrode sheets 2 can be directly distinguished.

A group of electrode sheets 2 preferably includes two to ten electrode sheets 2, and preferably includes three electrode sheets 2. After the stimulation electrode is unfolded, the number of electrode sheets 2 in a group of electrode sheets 2 is maintained at two to ten, which can meet most stimulation needs for neural electrical stimulation. The more electrode sheets 2 are in a group of electrode sheets 2, the more accurate the stimulation can be applied. For example, when the number of electrode sheets 2 in a group of electrode sheets 2 reaches ten, multiple electrode sheets 2 on the same stimulation electrode can accurately stimulate corresponding to-be-stimulated points respectively, preventing the poor treatment effect due to different intensities of stimulation required by multiple to-be-stimulated points when one electrode sheet 2 is used for stimulating the multiple to-be-stimulated points.

In another embodiment, in a group of electrode sheets 2, the local thickness of at least one electrode sheet 2 is different from the thickness of the other part of the at least one electrode sheet 2. Under the imaging technique, if the local thickness of the same electrode sheet 2 is less than the thickness of the other part of the same electrode sheet 2, the color of the thinner position is lighter than the color of the thicker position. Moreover, different electrodes 2 may also be distinguished from each other by different shades of color during imaging. In this manner, distinguishing between the positions of electrode sheets 2 in a group of electrode sheets 2 is combined with distinguishing between electrode sheets 2 through shapes, so the electrode orientation can be marked and recognized more accurately, and the position and direction for implanting the sliced stimulation electrode 3 can be determined accurately.

The present application further provides a stimulation system. The stimulation system includes a stimulator, a wire and a stimulation electrode, where the stimulator is connected to the stimulation electrode through the wire, and the stimulation electrode is the preceding sliced stimulation electrode 3. When the stimulation system is in use, the stimulator emits a signal, the signal is transmitted through the wire to the connecting end of the sliced stimulation electrode 3 and then is transmitted through the connecting end of the sliced stimulation electrode 3 and the electrode lead 1 to the electrode sheet 2 on the stimulating end of the sliced stimulation electrode 3, the electrode sheet 2 of the stimulation electrode releases the signal to stimulate a specified part of the brain, and thus an image is generated by an imaging device such as an X-ray machine. Different electrode sheets 2 are distinguished from each other according to shapes and other features of the electrode sheets 2, so the specific positions of the electrode sheets 2 on the sliced stimulation electrode 3 are distinguished from each other, which can prevent the electrode sheets 2 of the sliced stimulation electrode 3 from touching parts that do not require stimulation and prevent the resulting damage caused by the sliced stimulation electrode 3 to other tissues of the patient.

Although the embodiments of the present application are shown and described above, it is to be understood that the preceding embodiments are exemplary and cannot be understood as limitations to the present application. Those of ordinary skill in the art can make changes, modifications, substitutions and deformations to the preceding embodiments within the scope of the present application without departing from the principles and purposes of the present application, and all of these changes should fall within the scope of the claims of the present application.

## Claims

1. A sliced stimulation electrode, comprising a stimulating end and a connecting end which are opposite to each other, wherein a plurality of electrode sheets for applying electrical stimulation are disposed on a periphery surface of the stimulating end of the sliced stimulation electrode;
electrode sheets of the plurality of electrode sheets and passing through a same radial plane of the sliced stimulation electrode are a group of electrode sheets; and
in at least one group of electrode sheets, at least one electrode sheet has a different shape from the other electrode sheets so that electrode sheets in the at least one group of electrode sheets are capable of being distinguished from each other.

2. The sliced stimulation electrode according to claim 1, wherein a shape of each electrode sheet of the plurality of electrode sheets is different, or a shape of each electrode sheet in the group of electrode sheets is different.

3. The sliced stimulation electrode according to claim 1, wherein in a flattened state of the stimulating end of the sliced stimulation electrode, the plurality of electrode sheets are arranged in an array and at intervals; and
a guide shape is formed on at least one electrode sheet in the group of electrode sheets, wherein the guide shape of the at least one electrode sheet is used for distinguishing between the electrode sheets in the group of electrode sheets.

4. The sliced stimulation electrode according to claim 3, wherein the guide shape of the at least one electrode sheet is located on a side edge of the at least one electrode sheet or two opposite side edges of the at least one electrode sheet.

5. The sliced stimulation electrode according to claim 4, wherein the guide shape of the at least one electrode sheet is formed by a wave, a sine curve, a semicircle, a rounded rectangle or a rounded triangle;
the guide shape of the at least one electrode sheet is smoothly connected to the at least one electrode sheet; and
a shape of a peripheral edge of each electrode sheet of the plurality of electrode sheets is a closed curve.

6. The sliced stimulation electrode according to claim 1, wherein a shape of a peripheral edge of each electrode sheet of the plurality of electrode sheets is a smooth contour curve.

7. The sliced stimulation electrode according to claim 1, wherein in a flattened state of the stimulating end of the sliced stimulation electrode, the plurality of electrode sheets are arranged in an array and at intervals; and
a row of electrode sheets of the plurality of electrode sheets in a length direction of the sliced stimulation electrode have a same shape.

8. The sliced stimulation electrode according to claim 1, wherein the group of electrode sheets comprises two to ten electrode sheets.

9. The sliced stimulation electrode according to claim 1, wherein in the group of electrode sheets, local thickness of at least one electrode sheet is different from thickness of the other part of the at least one electrode sheet.

10. A stimulation system, comprising a stimulator, a wire and a stimulation electrode, wherein the stimulator is connected to the stimulation electrode through the wire, and the stimulation electrode is the sliced stimulation electrode according to any one of claims 1 to 9.
